# EUROPEAN PATENT APPLICATION

(11) **EP 3 967 277 A1**
(43) Date of publication of application: **16.03.2022**
(21) Application number: 20195401.3
(22) Date of filing: 10.09.2020
(51) Int. Cl.: A61F 2/38, A61F 2/46, A61F 2/30, A61B 17/72

(54) **FEMORAL STEM OFFSET ADJUSTMENT SYSTEM**

(71) Applicant: Aesculap AG, 78532 Tuttlingen (DE)
(72) Inventor: Girouard, Christophe, 52000 Sarcicourt (FR)
(74) Representative: Winter, Brandl - Partnerschaft mbB

(57) **Abstract**

The invention relates to a femoral stem offset adjustment system (1) for adjusting a femur implant (2) in reference to an intramedullary channel (3) of a femur, the adjustment system (1) comprising a femur support (5), a user adjustment element (6), a stem support (7), a first link member (8) rotatably holding the user adjustment element (6) in the femur support (5), a second link member (9) integrally rotatably coupling the user adjustment element (6) and the stem support (7), and a third link member slidably connecting the user adjustment element (6) and the stem support (7), wherein at least two of the first link member (8), the second link member (9) and the third link member, preferably the longitudinal axes thereof, are arranged substantially in a common plane, the plane being perpendicular to the rotational axis.

## Description

The invention concerns joint arthroplasty, especially knee arthroplasty and total knee arthroplasty. More specifically, the invention concerns devices which enable the surgeon to efficiently and accurately adjust a femur implant in reference to an intramedullary channel of a femur. In particular, the invention relates to a femoral stem offset adjustment system/device/instrument (hereinafter also referred to as adjustment system or adjustment instrument).

### Background

The knee joint enables a person's leg to flex or articulate during movement. At the knee, a lower bone (tibia) meets an upper bone (femur). Proximate the knee, the femur has two projections known as femoral condyles. Femoral condyles engage fibrocartilage at the upper end of the tibia. The knee joint is held together by ligaments, capsule, muscle, and tendons. Four ligaments are especially prominent in knee structure, with one ligament on either side of the knee and two ligaments in the center. Of the center ligaments, one ligament is oriented toward the front and one toward the back. The patella or knee cap is a piece of bone supported in front of the knee joint. Functionally, the patella acts as a shield.

The knee joint may be rendered nearly or totally inoperative by extended and heavy use, disease, or trauma. Often, the best therapy is total replacement (arthroplasty). During total knee arthroplasty, the femoral and tibial surfaces joined at the knee are totally replaced. The first step in this process is the removal of the condylar surfaces and some underlying portions thereof. The distal end of the femur is resected to provide clearance for a femoral prosthetic component. Similarly, a proximal portion of the tibia is resected to provide a flat surface for a tibial prosthetic component. A person's weight is transferred from the head of the femur proximate the hip to the ankle. Proper alignment of the knee must be maintained when femoral and tibial prosthetic devices (implants) are installed to allow the proper transfer of weight to continue. In order for the prostheses to function properly, femoral and tibial surfaces arising from these resections as well as a femur implant and a tibia implant must be correctly aligned. Further, in order to render the knee stable in flexion and extension, the femur and the tibial implant have to be properly distanced apart.

Once a bone has been properly prepared to accept a prosthesis component, surgeons typically utilize a "trial" system of prosthesis components that are provided by the prosthesis manufacturer. The trial components are sample prosthesis components, available in various sizes and shapes, that are intended to be placed into the prepared bone on a temporary basis for evaluation the correct size and shape of the prosthesis as well as the correct alignment, adjustment and orientation in situ on the patient. For adjustment of an offset position of the (trial) femur component in reference to an intramedullary channel of the femur, a femoral stem offset adjustment system is used.

Further, the patient is subjected to a trial reduction using blocks/templates of varying thickness to evaluate the flexion gap and the extension gap between the femur implant and the tibia implant. This trial reduction represents moving the joint, including the trial implant through selected typical motions for that joint. If the knee is unstable or misaligned, for example, a thicker or thinner tibial implant can be used.

In known femoral stem offset adjustment systems, the offset adjustment is performed in place, but - because of its structure and size - the adjustment system protrudes beyond the (trial) femur implant. In particular, since a rotational movement is usually combined with a unidirectional guided sliding movement to adjust the offset in any direction, the system requires a certain amount of installation space, especially in the axial direction, for the various relative movability of the connected parts. It is particularly disadvantageous, that the adjustment system has to be removed for correctly performing the flexion and extension gap assessment, because this removal is time consuming and means an additional work step.

Thus, there may be a need for an enhanced femoral stem offset adjustment system having a compact and reduced structure allowing the adjustment system to perform an offset adjustment as well as to dimension the extension and flexion gap while the adjustment system is mounted in situ on the patient, in order to enable the surgeon to quickly and efficiently align the (trial) femur implant.

### Short summary of the invention

This object is solved by an femoral stem offset adjustment system with the features of claim 1. Further advantageous developments of the present invention are made to the subject-matter of the respective sub claims.

The femoral stem offset adjustment system is for adjusting (orientating) a (trial) femur implant (prosthesis), in particular a lower extremity femur implant, in reference to (relative to) an intramedullary channel of a femur. The femoral stem offset adjustment system is in particular for adjusting an offset position of the femur implant relative to a (trial) stem corresponding to the intramedullary channel.

The adjustment system comprises a femur support. The femur support is adapted to be (fixedly/statically/non-rotationally and undisplaceable) connected to the femur implant. For example, the femur support may be fixedly attached to the femur implant by a femur attachment element, such as a screw, pin or the like.

Further, the adjustment system comprises a user adjustment element. The user adjustment element may be formed by a knob-like element, such as a wheel or the like. The user adjustment element is rotatably held/received by the femur support around a rotational axis. That is, the user adjustment element is attached to the femur support such that the femur support and the user adjustment element are kept rotatable to each other (the user adjustment element is connected to the femur support so as to rotate around a rotational axis relative to the femur support). Preferably, the rotational axis corresponds to a longitudinal axis of the adjustment system / to the axis of the intramedullary channel. Thus, it is possible to set an offset direction (clock position) of the femur support (and thus the femur implant) relative to the user adjustment element.

Further, the adjustment system comprises a stem support. The stem support is adapted to be (fixedly/statically/non-rotationally and undisplaceable) connected to the intramedullary channel. In particular, the stem support is adapted to be connected to the stem corresponding to the (femur) intramedullary channel. The stem support is integrally rotatably coupled to the user adjustment element. That is, the stem support is attached to the user adjustment element such that the stem support and the user adjustment element are rotationally linked to each other (the stem support is connected to the user adjustment element so as to integrally rotate with the user adjustment element). Further, the stem support is slidably received by/connected to the user adjustment element along a sliding axis. Preferably, the stem support is unidirectionally slidably received by the user adjustment element. That is, the stem support is connected to the user adjustment element so as to be slidable along a sliding axis relative to the user adjustment element. The sliding axis is perpendicular to the rotational axis (and thus, perpendicular to the longitudinal axis of the adjustment system). Thus, it is possible to set an offset amount of the stem support (and thus the stem) relative to the user adjustment element. Since the femur support is freely rotational to the user adjustment element, the sliding axis along which the user adjustment element and the stem support are slidable to each other rotates with the relative rotation between the user adjustment element and the femur support, such that the combination of the relative rotation and the unidirectionally relative sliding may be converted to adjust an offset position between the femur support and the stem support in any direction.

In other words, the femoral stem offset adjustment system functionally connects the femur implant to the stem such that an offset direction and an offset amount can be adjusted in order to adjust the offset position. The offset direction is set by rotating the femur support relative to the user adjustment element (with the stem support) around the rotational axis. The offset amount is set by sliding the user adjustment element (with the femur support) relative to the stem support along the sliding axis which is perpendicular to the rotational axis. Hence, the femoral stem offset adjustment system provides a sliding adjustment along the sliding axis, wherein the sliding axis can be rotated around the rotational axis. Thus, the femur implant can be displaced to the stem in any direction perpendicular to the rotational axis.

According to the invention, the femoral stem offset adjustment system comprises a first link member / part rotatably holding the user adjustment element in the femur support, a second link member /part rotatably coupling the user adjustment element and the stem support and a third link member / part slidably connecting the user adjustment element and the stem support. At least two of the first link member, the second link member and the third link member, preferably the longitudinal axes thereof, are arranged substantially in a common plane. The common plane is perpendicular to the rotational axis. Substantially in a common plane means not only an exact arrangement within the plane and not only a tolerance range of the plane, but an arrangement within a common axial range, which may extend between 0 mm (exact arrangement in the plane) up to 10 mm (slight axial offset between at least two of the first link member, the second link member and the third link member), preferably up to 5 mm (minor axial offset between at least two of the first link member, the second link member and the third link member) along the rotational axis.

In other words, two or all of (the longitudinal axes of) the first link member, the second link member and the third link member are arranged in a (common) plane. Due to the arrangement in the same plane of the link members the femoral stem offset adjustment system has a reduced length, in the direction perpendicular to the plane, that is, along the rotational axis/longitudinal direction, and an axially compact design. The reduced size of the femoral stem offset adjustment system allows it to be integrated into the (trial) femur implant, in particular into the thickness of the (trial) femur implant, such that the adjustment system does not protrude beyond the femur implant. Hence, the extension and flexion gap trial reduction can be performed with the adjustment system in place. This has the advantage that the surgeon can perform the adjustment in a time saving manner.

In short, according to the invention at least two of the first link member, the second link member and the third link member (providing the functional connection between the femur support, the user adjustment element and the stem support) are arranged radially spaced/nested/stacked instead of axially spaced as proposed by the prior art. Due to the integration essentially in the same radial plane (that is, within the same axial range) an adjustment instrument being axially short can be provided.

According to a preferred embodiment the first link member and the second link member may be integrally formed by a fastening element. In other words, the first link member and the second link member may be formed by the same component. That is, the fastening element enables the user adjustment element to be freely rotatable relative to the femur support as well as to be integrally rotatably connected to the stem support. Due to the integral design of the two functions (of the first and the second link member), a reduced number of components is required. Further, because the first and the second link member are formed by the same component, the longitudinal axes are identical and thus necessarily arranged in the same plane, which means the reduction of the axial design of the adjustment system, as described above.

According to the preferred embodiment, a radially outer portion of the fastening element may form the first link member and a radially inner portion of the fastening element may form the second link member. Thus, a radially stacked arrangement of the link members can be provided in order to reduce the axial length of the adjustment system. In other words, the fastening element is arranged radially between the femur support and the user adjustment element (connecting them freely rotatable to each other) as well as radially between the user adjustment element and the stem support (connected them non-rotatable to each other).

According to the preferred embodiment, the fastening element may be formed by two pins. The pins may be arranged coaxially to each other. The pins may be arranged axially spaced apart. Preferably, the pins may be arranged on opposite sides of the stem support (in the circumferential direction), in particular contacting the stem support. The circumferentially opposite arrangement is advantageous because (additional to the prevention of the relative rotation) a (translational) movement of the stem support in a radial direction (that is, perpendicular to the circumferential direction) relative to the pins (and thus relative to the user adjustment element) is prevented.

According to a preferred embodiment, the first link member may engage with a circumferential groove in the femur support. Preferably, the circumferential groove may be formed in the inner circumferential surface of the femur support. The circumferential groove may be a 360° circumferential groove in order to provide 360° free turning/rotating of the user adjustment element relative to the femur support. Preferably, the first link member may engage with the circumferential groove such that the user adjustment element and the femur support are translationally coupled to each other.

According to a preferred embodiment, the second link member may engage with/contact the outer peripheral surface of the stem support. The outer peripheral surface may be perpendicular to the longitudinal axis of the second link member. Thus, a translation of the stem support relative to the user adjustment element in a direction along the outer peripheral surface, that is, in a direction perpendicular to the longitudinal axis of the second link member, remains possible. In other words, the second link member engages with the outer peripheral surface of the stem support such that a sliding movement is allowed. The second link member may preferably engage axially beyond an axial end surface of the stem support. Thus, only a translation in a direction which is perpendicular to the axial direction/rotational axis and perpendicular to the radial direction remains possible. Hence, an unidirectional movement along the sliding axis is guided by the engagement of the outer peripheral surface and the second link member (the fastening element). A single component, that is, the fastening element, enables all necessary relative movements as well as fixes the components (user adjustment element, stem support, femur support) to each other (stops all other relative movements of the components).

According to a preferred embodiment, the third link member may be formed by a first sliding portion of the stem support and a sliding guiding portion of the user adjustment element receiving the first sliding portion. The sliding guiding portion and/or the first sliding portion may have an oblong shape elongated in the direction of the sliding axis. Thus, the sliding movement can be guided by positive locking of the portions. Preferably, the first sliding portion is arranged distally to the outer peripheral surface. Further, preferably, the sliding guiding portion is arranged distally to the fastening element.

According to a preferred embodiment, the third link member may be formed by a second sliding portion of the stem support and the fastening element, in particular the radial inner portion of the fastening element. The second sliding guiding portion may have an oblong shape elongated in the direction of the sliding axis. The second sliding guiding portion may form the outer peripheral surface such that the radial inner portion of the fastening element is guided by contacting the outer peripheral surface, allowing a sliding movement along the sliding axis. Thus, the sliding movement can be guided by positive locking of the portions. Further, due to the integral design of the second sliding guiding portion and the outer peripheral surface a compact configuration of the adjustment system can be provided.

According to a preferred embodiment, the femoral stem offset adjustment system may comprise an offset direction scale indicating the adjusted rotational position of the user adjustment element relative to the femur support. For example, the offset direction scale may be formed by numbers, in particular equal distanced, on an outer circumferential surface of the user adjustment element. Further, the offset direction scale may be formed by a reference mark on the femur support, in particular on a circumferential surface of the femur support. Thus, it is possible to easily place a final implant in the same offset direction as the trial femur implant.

According to a preferred embodiment, the femoral stem offset adjustment system may comprise an offset amount scale indicating the adjusted sliding position of the user adjustment element relative to the stem support. Thus, it is possible to easily place a final implant offset by the same offset amount as the trial femur implant. According to the preferred embodiment, the offset amount scale may be formed by a window in the user adjustment element and an offset mark on an (axial) end surface of the stem support.

According to a preferred embodiment, the femoral stem offset adjustment system may further comprise a femur implant connected to the femur support. An axial end surface of the user adjustment element may be proximal to an axial end surface of the femur implant. That is, the axial end surface of the user adjustment is recessed in the axial direction/in the longitudinal direction relative to the femur implant. This allows the adjustment instrument to remain mounted while assessing the extension gap and the flexion gap.

In other words, the purpose of this invention is to have a femoral stem offset adjustment system that allows to perform a flexion and extension gap with the instrument in place. The goal of the instrument is to place the femur implant in the best position in reference to the intramedullary channel. The adjustment system allows this positioning thanks to a 360° free turning adjustment system with an additional offset from 0 to 5 mm. The design of the adjustment system additionally allows to perform the flexion gap and the extension gap trial reduction with the adjustment system in place. This is due to the design of the instrument allowing it to be integrated into the thickness of the trial femur.

The adjustment system comprises a user adjustment element (wheel) which is the turning part that indicates the clock position of the instrument when it is well placed in the femur intramedullary channel. This user adjustment element is free of rotation inside a femur support thanks to two pins in the wheel and a groove in the femur support. The adjustment system comprises the (trial) femur support which is the part that allows the fixation of the adjustment system on the trial femur. The adjustment system comprises the (trial) stem support which is the part that can be connected to a (trial) stem. The (trial) stem support is linked to the wheel in rotation (360° free) and additionally allows a translation from 0 to 5 mm (offset) thanks to an oblong shape on the both parts. The offset value is indicated in a window of the wheel. The internal part of the pins fixed in the wheel allows to join the both parts (trial stem support and wheel) while allowing translation. The external part of the pins fixed in the wheel allows a free turning inside the femur support thanks to the internal groove of the femur support. The oblong shape in both parts (trial stem support and wheel) allows the translation (offset) between wheel and trial stem support.

Thus, the flexion and extension gap trial reduction can be performed thanks to a minimalist design of the adjustment system allowing to not overstep the thickness of the trial femur component. The dimensioning of the adjustment system allows to perform both flexion and extension gap having the main advantage to be able to perform the gap management without removing of the adjustment system, which is time saving and means an easier workflow.

### Brief Description of the Drawings

The invention is explained in more detail below on the basis of a preferred embodiment using figures. The figures are of a schematic nature and intended to improve the understanding of the invention. Same elements are referenced to with the same reference signs.
Figures 1 and 2 illustrate perspective exploded views of a femoral stem offset adjustment system,
Figure 3 illustrates a perspective view of the femoral stem offset adjustment system connected to a femur implant,
Figure 4 illustrates a perspective view of the femoral stem offset adjustment system,
Figures 5 to 10 illustrate perspective views explaining the use of the femoral stem offset adjustment system.

### Detailed Description of the Preferred Embodiment

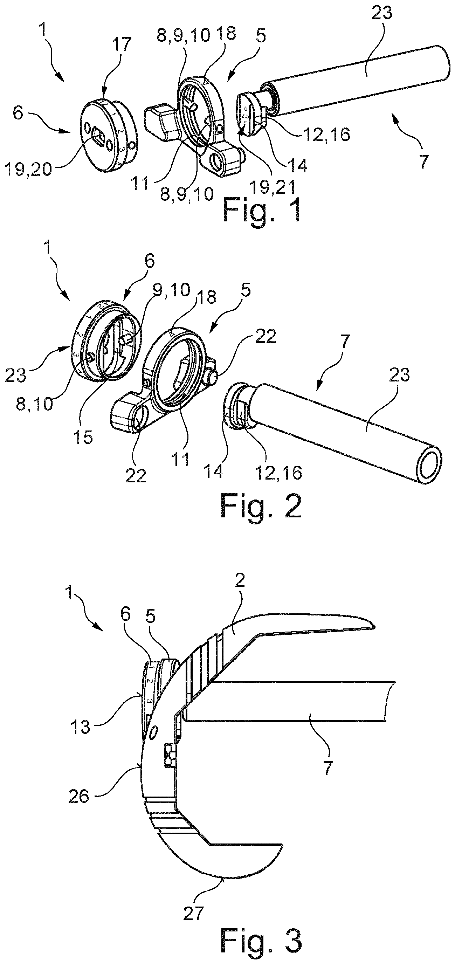

Figs. 1 to 4 show a preferred embodiment of a femoral stem offset adjustment system/instrument 1 (hereinafter referred to as adjustment system 1) according to the invention. The adjustment system 1 is for adjusting (orientating) a (trial) femur implant 2 (in particular, a lower extremity femur implant) relative to an intramedullary channel 3 of a femur. The adjustment system 1 guides the adjustment of an offset position of the femur implant 2 relative a (trial) stem 4 corresponding to the intramedullary channel 3 to achieve a desired posterior orientation of the femur implant 2.

The adjustment system 1 comprises a femur support 5. The femur support 5 is adapted to be connected to the femur implant 2. The femur support 5 attaches/fixes the femur support 5 statically to the femur implant 2. That is, the orientation of the femur implant 2 corresponds to the orientation of the femur support 5.

The adjustment system 1 comprises a user adjustment element 6. The user adjustment element 6 is distally connected to the femur support 5. The user adjustment element 6 is rotatably held by the femur support 5 around a rotational axis. The rotational axis corresponds to a longitudinal axis of the adjustment system 1. That is, the user adjustment element 6 is attached to the femur support 5 such that the femur support 5 and the user adjustment element 6 are kept rotatable to each other. The user adjustment element 6 is integrally translationally connected to the femur support 5. That is, the user adjustment element 6 is attached to the femur support 5 such that the femur support 5 and the user adjustment element 6 are translationally linked to each other (no relative displacement between the femur support 5 and the user adjustment element 6 is enabled).

The adjustment system 1 comprises a stem support 7. The stem support 7 is adapted to be connected to the intramedullary channel 3, in particular the stem 4. The stem support 7 attaches/fixes the stem support 7 statically to the stem 4. That is, the orientation of the stem 4 corresponds to the orientation of the stem support 7. The stem support 7 is proximally connected to the user adjustment element 6. The stem support 7 is integrally rotatably coupled to the user adjustment element 6. That is, the stem support 7 is attached to the user adjustment element 6 such that the stem support and the user adjustment element 6 are rotationally linked to each other (no relative rotation between the user adjustment element 6 and the stem support 7). The stem support 7 is slidably received by/connected to the user adjustment element 6 along a sliding axis. The sliding axis is perpendicular to the rotational axis. That is, the stem support 7 is attached to the user adjustment element 6 so as to be slidable along the sliding axis relative to the user adjustment element 6.

In other words, the adjustment system 1 functionally connects the femur support 5 (and thus, the femur implant 2) to the stem support 7 (and thus, the stem 4). Due to the combination of a rotation around the rotational axis and a sliding movement along the sliding axis (perpendicular to the rotational axis) of the user adjustment element 6 relative to the stem support 7, the adjustment system 1 is enabled adjust an offset translational movement of the femur support 5 relative to the stem support 7 along any desired direction perpendicular to the rotational axis (that is, any offset direction in the plane perpendicular to the rotational axis).

The adjustment system 1 comprises a first link member 8 attaching the femur support 5 and the user adjustment element 6 such that they are kept rotatable to each other. The adjustment system 1 comprises a second link member 9 attaching the user adjustment element 6 and the stem support 7 such that they are rotationally fixed to each other. The adjustment system 1 comprises a third link member slidably connecting the user adjustment element 6 and the stem support 7

At least two of the first link member 8, the second link member 9 and the third link member, preferably the longitudinal axes thereof, are arranged substantially in a common plane, the plane being perpendicular to the rotational axis. Substantially in a common plane means not only an exact arrangement within the plane and not only a tolerance range of the plane, but an arrangement within a common axial range, which may extend between 0 mm (exact arrangement in the plane) up to 10 mm (slight axial offset between at least two of the first link member, the second link member and the third link member), preferably between 0 mm up to 5 mm (minor axial offset between at least two of the first link member, the second link member and the third link member) along the rotational axis.

In the preferred embodiment, illustrated in Figs. 1 to 4, a longitudinal axis of the first link member 8 and a longitudinal axis of the second link member 9 run in a common plane perpendicular to the longitudinal direction of the adjustment system 1. In particular, the first link member 8 and the second link member 9 are integrally formed by a fastener element 10. A radially outer portion of the fastener element 10 forms the first link member 8. A radially inner portion of the fastener element 10 forms the second link member 9.

The radially outer portion forming the first link member 8 engages with a circumferential groove 11 in the femur support 5. The circumferential groove 11 is formed in an inner circumferential surface of the femur support 5. The circumferential groove 11 is 360° circumferential providing 360° free turning of the user adjustment element 6. The first link member 8 engages with the circumferential groove 11 such that the user adjustment element 6 and the femur support 5 are translationally coupled to each other.

The radially inner portion forming the second link member 9 engages with (contacts) an outer peripheral surface 12 of the stem support 7. The second link member 9 engages with the outer peripheral surface 12 such that the user adjustment element 6 and the stem support 7 are rotationally coupled to each other. The outer peripheral surface 12 is perpendicular to the longitudinal axis of the second link member 9. Thus, the user adjustment element 6 and the stem support 7 are freely translationally slidable along the outer peripheral surface 12. The outer peripheral surface 12 is proximal to an axial end surface 13 of the stem support 7. The second link member 6 protrudes radial inwards such that it engages proximally/axially behind the axial end surface 13. Thus, the user adjustment element 6 and the stem support 7 are coupled to each other in the axial direction corresponding to the longitudinal axis.

The fastening element 10 is formed by two pins. The pins are arranged coaxially to each other. The pins are spaced axially apart. The pins are arranged on opposite sides in the circumferential direction. The pins each passes through a hole in the user adjustment element 6. The pins are arranged on opposite sides of the stem support 7 each contacting an outer peripheral surface 12 of the stem support 7. That is, the translational movement of the stem support 7 is guided by the two pins.

The third link member is formed by a first sliding portion 14 of the stem support 7 and a sliding guiding portion 15 of the user adjustment element 6. The first sliding portion 14 has an oblong shape elongated in the direction of the sliding axis. The first sliding portion 14 is received by the corresponding sliding guiding portion 15 of the user adjustment element 6. The sliding guiding portion 15 has an oblong shape elongated in the direction of the sliding axis. The first sliding portion 14 and the sliding guiding portion 15 attach the adjustment element 6 and the stem support 7 such that they are kept slidable to each other along the sliding axis.

The third link member is formed by a second sliding portion 16 of the stem support 7 and the fastening element 10. The second sliding portion 16 has an oblong shape elongated in the direction of the sliding axis. The second sliding portion 16 is forming the outer peripheral surface 12. The outer peripheral surface 12 engages with/ contacts the fastening element 10, in particular the radially inner portion of the fastening element 10. The second sliding portion 16 and the fastening element 10 attach the adjustment element 6 and the stem support 7 such that they are kept slidable to each other along the sliding axis.

The adjustment instrument 1 comprises an offset direction scale 17 indicating the adjusted rotational position of the user adjustment element 6 relative to the femur support 5. The offset direction scale 17 is formed by numbers on an outer circumferential surface of the user adjustment element 6 indicating a clock position of the user adjustment element 6 relative to a reference mark 18 on an outer circumferential surface of the femur support. The rotational position of the user adjustment element 6 relative to the femur support 5 may be adjusted 360°.

The adjustment instrument 1 comprises an offset amount scale 19 indicating the adjusted sliding position of the user adjustment element 6 relative to the stem support 7. The offset amount scale 19 formed by a window 20 in the user adjustment element 6, in particular in the axial end surface 13, and an offset mark 21 on an end surface of the stem support 7. The sliding position of the user adjustment element 6 relative to the stem support 7 may be adjusted from 0 to 5 mm.

The femur support 5 comprises an femur attachment member 22 fixedly connecting the femur implant 2 to the femur support 5. The stem support 7 comprises a stem attachment member 23 fixedly connecting the stem 4 to the stem support 7.

Fig. 3 illustrates the femur implant 2 mounted to the adjustment instrument 1. The femur implant 2 comprises an extension end surface 26 relative to which an extension gap between tibia and femur is set. The femur implant 2 comprises a flexion end surface 27 relative to which a flexion gap between tibia and femur is set. It can be seen, that the axial end surface 13 does not protrude axially beyond the extension end surface 26 (nor radially beyond the flexion end surface 27). Thus, the flexion gap and the extension gap can be assessed with the adjustment instrument 1 in place.

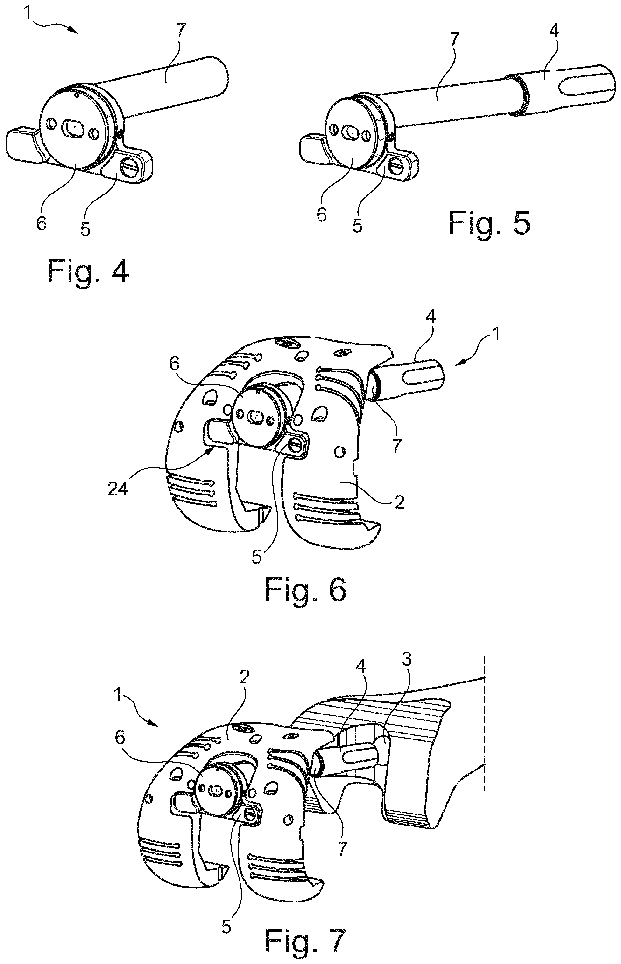

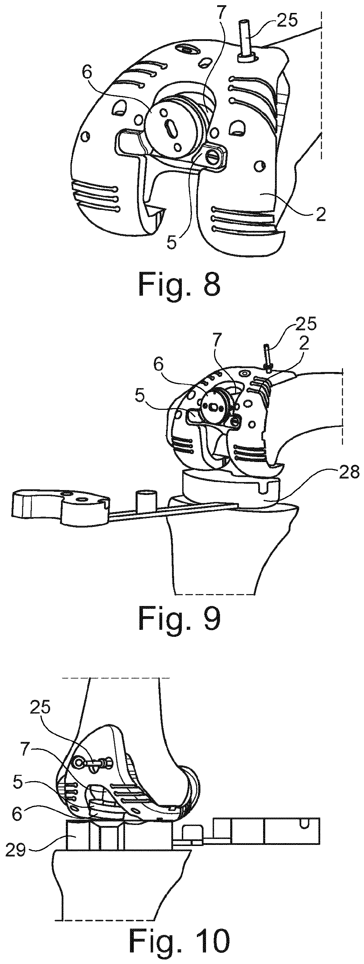

Figs. 4 to 10 illustrate the use of the adjustment instrument 1. Fig. 4 illustrates the adjustment instrument 1 before mounting the femur implant 2 and the stem 4. In a first step (Fig. 5), the adjustment instrument 1 (the stem support 7) is connected to the (trial) stem 4 corresponding to the femur intramedullary channel 3. The stem attachment member 23 is inserted to the stem 4. In a second step (Fig. 6), the adjustment instrument 1 (the femur support 5) is connected to the (trial) femur implant 2. The femur attachment member 22 engages with a corresponding recess 24 in the femur implant 2. In a third step (Fig. 7), the adjustment instrument 1 (the stem 4) is inserted into the femur intramedullary channel 3. In a fourth step (Fig. 8), femur implant 2 is placed to fit on the femur by adjusting the offset direction (by rotating the user adjustment element 6 relative to the femur support 5) and the offset amount (by sliding the user adjustment element 6 relative to the stem support 7). The femur implant 2 is attached to the femur by a femur fastening element 25. Since the adjustment instrument 1 does not project beyond the surfaces of the femur implant 2, in particular the extension end surface 26 (or the flexion end surface 27), the flexion gap trial reduction and the extension gap trial reduction can be performed while the adjustment instrument 1 is mounted to the femur (without removing the adjustment instrument 1). For the flexion gap trial reduction (Fig. 9), a flexion distance template 28 is placed contacting the surface of the tibia and the flexion end surface 27. For the extension gap trial reduction (Fig. 10), an extension distance template 29 is placed contacting the surface of the tibia and the extension end surface 26.

The offset amount and the offset direction are indicated on the offset direction scale 17 and the offset amount scale 19 of the adjustment instrument 1. Thus, a final implant can be placed in the same position/orientation as the (trial) femur implant 2.

## Claims

1. Femoral stem offset adjustment system (1) for adjusting a femur implant (2) in reference to an intramedullary channel (3) of a femur, in particular for adjusting an offset position of the femur implant relative to a stem (4) corresponding to the intramedullary channel (3), the adjustment system (1) comprising a femur support (5), the femur support (5) being adapted to be connected to the femur implant (2), a user adjustment element (6), the user adjustment element being rotatably held by the femur support (2) around a rotational axis, the rotational axis preferably corresponding to a longitudinal axis of the intramedullary channel (3), and a stem support (7), the stem support (7) being adapted to be connected to the intramedullary channel (3), in particular to the stem (4) corresponding to the intramedullary channel (3), being integrally rotatably coupled and being slidably connected to the user adjustment element (6) along a sliding axis, the sliding axis being perpendicular to the rotational axis,
**characterized by** a first link member (8) rotatably holding the user adjustment element (6) in the femur support (5), a second link member (9) integrally rotatably coupling the user adjustment element (6) and the stem support (7), and a third link member slidably connecting the user adjustment element (6) and the stem support (7), wherein at least two of the first link member (8), the second link member (9) and the third link member, preferably the longitudinal axes thereof, are arranged substantially in a common plane, the plane being perpendicular to the rotational axis.

2. Femoral stem offset adjustment system (1) according to claim 1, wherein the first link member (8) and the second link member (9) are integrally formed by a fastening element (10).

3. Femoral stem offset adjustment system (1) according to claim 2, wherein a radially outer portion of the fastening element (10) forms the first link member (8) and a radially inner portion of the fastening element (10) forms the second link member (9).

4. Femoral stem offset adjustment system (1) according to claim 2 or 3, wherein the fastening element (10) is formed by two pins, the pins being arranged coaxially and spaced axially apart, preferably on opposite sides of the stem support (7).

5. Femoral stem offset adjustment system (1) according to any of claims 1 to 4, wherein the first link member (8) engages with a circumferential groove (11) in the femur support (5), preferably on the inner circumferential surface of the femur support (5).

6. Femoral stem offset adjustment system (1) according to any of claims 1 to 5, wherein the second link member engages (9) with an outer peripheral surface (12) of the stem support (7), the outer peripheral surface (12) being perpendicular to the longitudinal axis of the second link member (9), the second link member (9) preferably engaging axially beyond an axial end surface of the stem support (7).

7. Femoral stem offset adjustment system (1) according to any of claims 1 to 6, wherein the third link member is formed by a first sliding portion (14) of the stem support (7) and a sliding guiding portion (15) of the user adjustment element (6) receiving the first sliding portion (14), the sliding guiding portion (15) and/or the first sliding portion (14) having an oblong shape elongated in the direction of the sliding axis.

8. Femoral stem offset adjustment system (1) according to claims 6 or 7, wherein the third link member is formed by a second sliding portion (16) of the stem support (7) and the fastening element (10), the second sliding guiding portion (16) having an oblong shape elongated in the direction of the sliding axis and forming the outer peripheral surface (12).

9. Femoral stem offset adjustment system (1) according to any of claims 1 to 8, wherein the femoral stem offset adjustment system (1) comprises an offset direction scale (17) indicating the adjusted rotational position of the user adjustment element (6) relative to the femur support (5), the offset direction scale (17) preferably formed by numbers (17) on an outer circumferential surface of the user adjustment element (6) and a reference mark (18) on a circumferential surface of the femur support (5).

10. Femoral stem offset adjustment system (1) according to any of claims 1 to 9, wherein the femoral stem offset adjustment system (1) comprises an offset amount scale (19) indicating the adjusted sliding position of the user adjustment element (6) relative to the stem support (7), the offset amount scale (19) preferably being formed by a window (20) in the user adjustment element (6) and an offset mark (21) on an end surface of the stem support (7).
